# EUROPEAN PATENT SPECIFICATION

(11) **EP 2 816 143 B1**
(45) Date of publication and mention of the grant of the patent: **13.09.2017**
(21) Application number: 14001929.0
(22) Date of filing: 04.06.2014
(51) Int. Cl.: C25D 5/02, C25D 5/10, C25D 5/34, C25D 21/12, C30B 7/00, C30B 7/12, C30B 30/02

(54) **Process of producing a porous layer structure, product obtained and use of such product**
Verfahren zur Herstellung einer porösen Schichtstruktur, hergestelltes Produkt sowie dessen Verwendung
Procédé de production d'une structure de couche poreuse, produit obtenu et utilisation de ce produit

(30) Priority: 17.06.2013 EP 13003091
(43) Date of publication of application: 24.12.2014
(73) Proprietor: Hochschule Furtwangen, 78120 Furtwangen im Schwarzwald (DE)
(72) Inventor: Mozaffari-Jovein, Hadi, 78652 Deißlingen (DE); Soares, Tiago Augusto, 78532 Tuttlingen (DE)
(74) Representative: Müller-Boré & Partner Patentanwälte PartG mbB

(56) References cited:
- GB-A- 2 489 458
- T A Soares ET AL: "Kinetic Investigation of a Mechanism for Generating Microstructures on Polycrystalline Substrates Using an Electroplating Process Hochschule Furtwangen (Campus Tuttlingen)", , 10 October 2012 (2012-10-10), XP055089397, Retrieved from the Internet: URL:http://www.comsol.fi/papers/13269/down load/soares_paper.pdf [retrieved on 2013-11-20]

## Description

The present invention relates to the generation of microstructured patterns on a heterogeneous polycrystalline substrate to create a porous layer on said substrate. This generated porous layer serves to change the chemical and physical functions or properties of the substrate's surface. For instance, it can increase the catalytic properties of gas sensors, the biocompatibility of implant parts. The generated porous layer can also develop a metallic superhydrophobic behaviour of the surface. Approaches to generate porous microstructures on polycrystalline substrates are known in the art. For example, porous microstructures can be produced by means of complex and expensive processes like lithographic processes or laser techniques. In all these techniques, the respective porous layer is created by shaping the substrate with external tools.

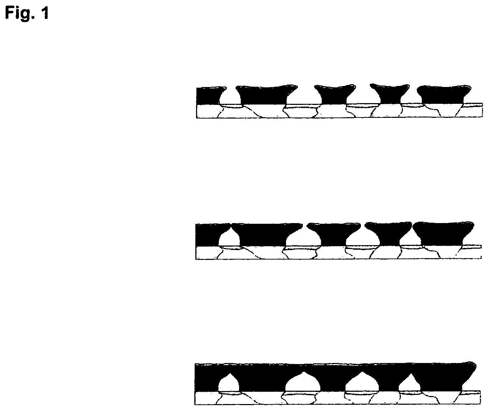

In an excerpt from the Proceedings of the 2012 COMSOL Conference in Milan, SOARES T, MOZAFFARI H, REINECKE H, Kinetic investigation of a mechanism for generating microstructures on polycrystalline substrates using an electroplating process, the generation of patterned microstructures on commercial metal polycrystalline substrates has been reported. Here, the pattern was defined by conducting a selective etching process on a two-phase polycrystalline substrate. Because of the difference in the chemical-physical properties of the crystal phases, the etching rate between the alpha and beta crystals were also different. As a result of this process, there were etched regions correspondent to beta-phase crystals and quasi non-etched regions that belong to alpha-phase crystals. Afterwards, the entire surface was covered with a polymer coat, and after drying, a polishing process was conducted in order to develop the pre-defined pattern, consisting of conductive and electrically insulated regions, which are confined to the crystal dimensions. Finally, microstructure patterns were generated through electrodeposition. However, in the course of the electrodeposition step the structures started to narrow their cavities until sealing the entire surface. This phenomenon is shown in Figure 1.

In view of the above, the technical problem underlying the present invention is to provide a process being effective in generating microstructured patterns on a heterogeneous polycrystalline substrate to create a porous layer on said substrate, without the problem of narrowing and sealing the substrate's entire surface, thereby affecting the physical-chemical properties of the substrate's surface.

According to the present invention, the above-described technical problem is solved by providing a process of producing a porous layer structure on a heterogeneous polycrystalline substrate, comprising the following steps:
(i) providing a heterogeneous polycrystalline substrate having at least two regions with different chemical-physical properties,
(ii) conducting a selective etching process on said polycrystalline substrate, resulting in etched regions corresponding to one of the at least two regions of said substrate and non-etched regions corresponding to the other one of said at least two regions of said substrate,
(iii) covering the entire surface of the substrate with an insulating material, thus creating an insulating layer on said substrate,
(iv) polishing the surface of the substrate to such an extent that the non-etched regions of step (ii) are exposed,
(v) depositing microstructures on said non-etched regions and stopping the deposition before the growth of the microstructures starts narrowing their cavities formed between adjacently growing microstructures, the depositing of the microstructures is performed by electroplating, cathodic or anodic polarization or electrophoresis, preferably by electroplating,
(vi) optionally washing the entire substrate having the microstructures deposited thereon with a solvent for removing the insulating material, and
(vii) repeating successively above steps (iii), (iv), (v) and (vi) in a loop process till a desired height of the microstructures is reached, where at least in the final loop the washing step (vi) is carried out.

According to the present invention, the production method mainly comprises 5 processing steps. In step (ii), a selective etching process is conducted at some specific areas of the surface of the polycrystalline substrate. These areas have particular chemical-physical properties, which could be different crystal structures or phases, changes in the composition, grain boundaries or other defects on the crystal structure. In step (iii), an electrically insulated layer, composed of organic or inorganic materials, is used to fill the areas of the polycrystalline substrate that were etched in step (ii). One of the ways of filling these areas consists in covering the whole surface of the etched polycrystalline substrate with the electrically insulated layer. As the insulating material, an organic polymeric resin can usually be used. Typically, a commonly known metal protective lacquer, usually an epoxy lacquer, a commonly known paint used in waterproof permanent marker pen (cf. for example US 7,084, 191 B1), a photoresist like for example SU-9 (epoxy based photoresist), or plating resist inks such as those available under the tradename NAZDAR can be employed. Then, a polishing step (iv) is conducted on the surface for removing the electrically insulated layer, up until exposing the regions that were not etched in step (ii). Afterwards, in step (v), the microstructures are deposited on the non-etched regions. This deposition could be done by electroplating, cathodic or anodic polarization, electrophoresis. The deposition is carried out until the growth of the microstructure starts narrowing their cavities and sealing the openings of the formed layer, i.e. the deposition is stopped before the growth of the microstructures starts narrowing their cavities formed between adjacently growing microstructures. The stop point of the deposition process depends mainly on the average of the grain's width. The concentration and the composition of the electrolyte, the stirring conditions, the surface roughness, the applied voltage and the temperature, can also have an influence during the deposition process. A good estimation of the stop point process could be calculated by the following relation: SP = (5* GW) + 66. Where: SP is the stop point in seconds and GW is the average of the grain's width in micrometer. For instance, for an average grain's width of 30 µm, the stop point would be 216 s. Moreover, the height of the formed microstructures will be approximately 15 µm. It can also be estimated by the relation: MH = GW / 2, where: MH is the microstructure height in micrometers. These values are true for an electrolyte concentration of: 200 mol/m³ of CuSO₄, diffusion coefficient of all species: 1.75 x 10⁻⁹, surface roughness of: Rq = 1206.8 °A, Sm = 12.2 µm, applied voltage of: 400 mV and the temperature of: 303 K.

In the optional step (vi) the entire substrate having the microstructures deposited thereon is washed with a solvent in order to remove the insulating material. The solvent can for example be an organic solvent like acetone. Other organic solvents like DMF, DMSO, THF, etc. can also be used.

In the final step (vii) the above steps (iii), (iv), (v) and (vi) are repeated in a loop process until the desired height of the produced porous layer is reached. At least in the final loop the washing step (vi) is mandatorily carried out to get the porous structure. In each step of the loop, the height of the structure will grow approximately 15 µm (for the conditions mentioned above), for running the loop, for instance, 10 times, the microstructures would have 150 µm, thereby obtaining a porous layer structure on said heterogeneous polycrystalline substrate. Shorter or smaller porous layer could be active by controlling the size of the grains or the deposition time or the number of the loops.

Thus, basically, the developed process consists of the consecutive steps: 1. Etching, 2. Patterning, 3. Deposition, 4. Washing (which is optional depending on the insulating material used) and 5. Loop process (including a final washing step to get the porous structure).

In a preferred embodiment of the present invention, the at least two regions of said heterogeneous polycrystalline substrate are different in crystal structure, crystal phase or chemical composition. For instance, in Cu-alloys, Ti-alloys, Fe-alloys, Al-alloys, Mg-alloys, it is possible to have two or more different crystal structures in equilibrium in the same substrate. For example, in CuZn₃₇ system, the alpha crystal would have approximately 36% Zn and the beta crystal would have approximately 44% Zn. In a more preferred embodiment, the process of the present invention comprises the steps of (i) providing a heterogeneous polycrystalline substrate having two or more phases in thermodynamic equilibrium, and (ii) conducting a selective etching process on said two or more phases polycrystalline substrate, resulting in etched regions corresponding to one or more phases thereof and non-etched regions corresponding to the other phases. Such polycrystalline substrates usable in the present invention can be selected from two or more phase's substrate. From metallurgy principles, when two or more materials are mixed in an alloy, depending on the individual concentration of the mixed materials, it is possible to have one type of crystal structure (homogeneous mixture) or two or more types of crystal structures (heterogeneous mixture).

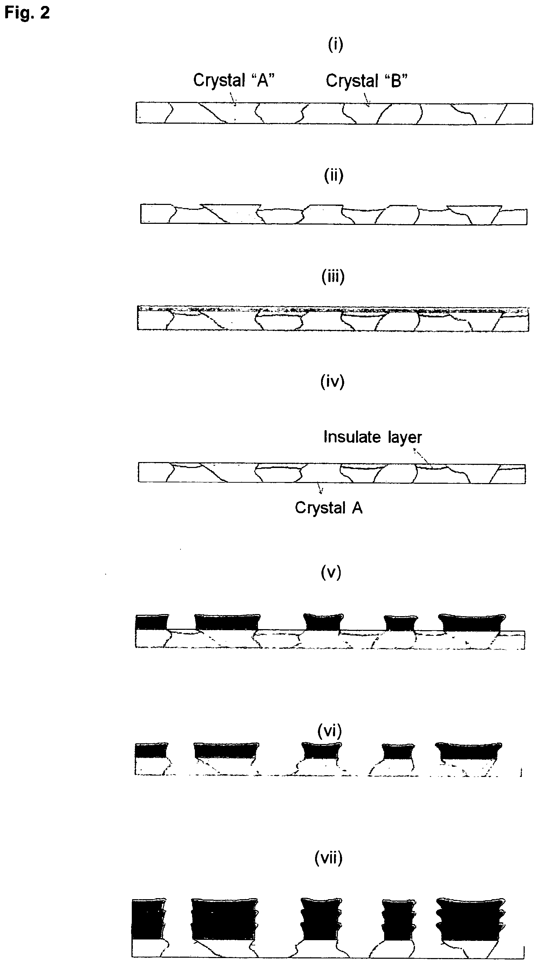

The present invention targets to use the properties of a polycrystalline substrate for creating a so-called "seed porous" on the surface and filling it later with an insulating material. Figure 2 schematically shows the whole process according to the present invention.

Prior to the actually adopted process steps, at first, there is provided a heterogeneous polycrystalline substrate. Most of industrial alloys are composed of polycrystalline structures. These structures are a group of crystalline grains, with different shapes and atomic orientations. Depending on the composition of these solid materials, it is possible to have two or more phases in thermodynamic equilibrium, and this is called a heterogeneous solution. Here, in Figure 2, crystals A and B can be standard structures of a chosen metal like Cu-alloy. In particular, a CuZn₃₇ system can be used as an electrode material. At this composition (37% Zn and 63% Cu), two phases (α+β), or a mixture of two crystal structures (FCC - Face-centered cubic and BCC - Body-centered cubic) can be in equilibrium. If the system is in equilibrium, the energy level of both phases is the same. However, looking at the surface, the difference between crystal structures and composition can considerably change the surface energy of one phase, in comparison to the other. In other words, the number of free bonds of atoms on the crystal surface, and the enthalpy of interaction between atoms are different.

In the following step (ii), selective etching is carried out. Preferably, this is done by wet etching, e.g. by using nitric acid. The result of the etching step can be taken from Figure 2(ii), namely one type of crystal is etched faster than the other. This leads to the creation of a so-called "seed porous". The porous arise from the exposed metal or metal alloy, respectively. After completion of step (iii) the "seed porous" corresponds to the regions where the metal (crystal) is not protected by the insulating layer.

In the above example of a CuZn₃₇ system, if the concentration of Zinc is higher on the β phase, the chance of dezincification in an acid solution can be higher. Additionally, due to the difference in corrosion potentials, a local redox reaction can occur spontaneously along the crystal phases, in which the β phase would work as an anode. For such a system, HNO₃ (65%) can be used for carrying out etching.

Then, in step (iii) the whole surface is filled with an insulating material. Typically, the insulating material is an organic resin, preferably applied on the entire surface of the substrate by means of coating. Particularly, the whole substrate is covered with a mixture of polymer resin, preferentially epoxy, and solvent and left to dry for a few minutes under standard conditions.

Afterwards, a polishing process (iv) is conducted in order to remove the polymer layer thus obtained, i.e. the substrate's surface is polished until exposing the crystal A. Preferably, polishing is carried by a polishing machine used in metallographic experiments. As a result, in the above example, there are isolated regions above the β-crystals and metal regions which belong to the α-crystals.

In the next step, depositing of micro-nanostructures on said non-etched regions till the growth of the microstructures starts narrowing their cavities formed between adjacently growing microstructures and, thus sealing the openings of said cavities, is carried out. In step (v) the depositing of the microstructures is performed by electroplating, cathodic or anodic polarization or electrophoresis, preferably by electroplating. In a preferred embodiment, particularly when using copper as the material to be deposited, the electroplating time is less than 300s. Particularly, it is 216 s for an average grain's width of 30 µm.

In the above example of a CuZn₃₇ system, electrodeposition can be conducted in a standard CuSO₄ electrolyte. A hot stirring plate is used in order to reasonably keep a constant temperature and a uniform agitation in the bath. The voltage is kept in a range of 350 mV to 450 mV, and the plating time is set up to 216 s.

Finally, as step (vii), repeating steps (iii), (iv), (v) and (vi) leads to the desired height. Typically, step (vii) is carried out as many times till an height of the microstructures in the range of 15 µm to 250 µm, preferably 100 µm to 250 µm, more preferably 100 µm to 200 µm, is reached. Regarding the height, it depends on how many times step (vii) is executed.

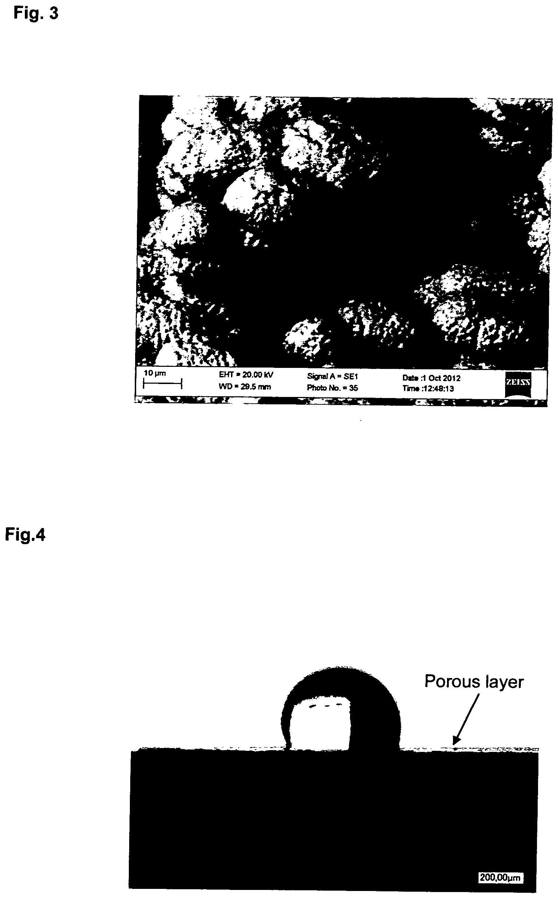

The advantage in increasing the height of the microstructure in such a loop process (vii) consists of, i.e., increasing the surface area of gas sensors, controlling the adhesion and friction of mechanical parts on biomedical industry, construction industry, sport and equipments. Additionally, super-hydrophobic property has surprisingly been found in the proposed porous layer (Fig. 4). Moreover, surfaces with higher microstructures, e.g. 150 µm, would have different functions and tribological interactions than microstructures with 15 µm in height. Retention of lubricant films and abrasive parts could be enhanced by higher microstructures.

The aforementioned etching step (ii) is important, insofar it creates the "seed porous". In an industrial production line, steps (iii), (iv) and (v) could be substituted by other convenient processes such as Screen Printer technology. In any case, there is no need to conduct again the etching step (ii) during the loop process (vii), because the "seed porous" is already there after completion of step (vii).

Stopping the deposition step before the growth of the microstructures starts narrowing their cavities formed between adjacently growing microstructures and repeating successively above steps (iii), (iv), (v) and (vi) in a loop process leads to the generation of the desired porous layer of patterned microstructures, thereby avoiding the "sealing" of the substrate's surface during the deposition step, preferably electroplating step. During the deposition step, the growth of the microstructure is not only on a vertical way, but also on the sides. According to the present invention, stopping this side effect and keeping the growth of the microstructures is achieved by performing steps (v), (vi) and (vii). Figure 1 exemplifies these "side effects".

In the process according to the present invention, the size of the "seed porous", i.e. the surface area on which the microstructures can be deposited in step (v), can be controlled by adjusting the size of the crystals. Thereby, the size of the crystals would be the size of the seed porous. One way of enlarging the size of the crystals is conducting a heat treatment. Experiments show the possibility of having crystal sizes from 1 µm up to 200 µm.

According to a preferred embodiment of the present invention, micro-fabrication of Cu structures can be performed by means of electrodeposition on heterogeneous polycrystalline substrate (α+β) crystals. As mentioned before, the height of the generated microstructures is basically defined by the size of the substrate's crystalline grains. Usually, these dimensions range from 1 µm up to 200 µm, particularly 30 µm. In accordance with the adopted strategy, part of the grains can have their surface protected with an insulate film (insulating layer), while the unprotected areas act as regions of nucleation and/or growth of Cu films.

Figure 3 shows the microscopic picture of thus created microstructures on CuZn₃₇ substrate, and pure Cu was electrodeposited. In this case, step (vii) has been executed 6 times.

Figure 4 shows the super hydrophobic properties (Lotus effect) on the thus generated porous layer.

The main advantage of the process according to the present invention resides in using the properties of standard industrial material to produce the porous layer in a very simple and cost-effective way. The physical effect for producing the "seed porous" is already on the thus provided material.

Another advantage is the random distribution of the size of the porous microstructures Usually, the generated microstructures have not exactly the same size. In the medical field, this random distribution could increase the biocompatibility of implant parts, since the size of the cells are not the same. In the medical field, biocompatibility of e.g. titanium implant parts or anti-bacterial effects of medical instruments or tools can be enhanced by providing such porous layers generated by the process according to the present invention. Additionally, super hydrophobic properties were also found on the developed metallic (Cu) porous layer. The porous layer has surprisingly shown a Lotus effect on the surface.

Moreover, such microstructures (porous layer) can be created on sensor materials to increase the contact area between the sensor and the reactant fluid. Such microstructures can accelerate the reactions in some catalytic processes and can also change the chemical energy of the substrate. For instance, copper oxide (CuO) is known as an element for sensing many gases including CO₂. Recently, it was reported the possibility of using CuO for sensing methanol and ethanol, and the presence of microporous structures is extremely important in the progress of such reaction. For example, in tropical countries, this effect could be used for detecting the amount of ethanol in "flex fuel cars" where ethanol and gasoline can be mixed.

Finally, the porous layer generated by the process according to the present invention can be used for decreasing the friction of mechanical parts through the retention of lubricant films.

## Claims

1. A process of producing a porous layer structure on a heterogeneous polycrystalline substrate, comprising the following steps:
(i) providing a heterogeneous polycrystalline substrate having at least two regions with different chemical-physical properties,
(ii) conducting a selective etching process on said polycrystalline substrate, resulting in etched regions corresponding to one of the at least two regions of said substrate and non-etched regions corresponding to the other one of said at least two regions of said substrate,
(iii) covering the entire surface of the substrate with an insulating material, thus creating an insulating layer on said substrate,
(iv) polishing the surface of the substrate to such an extent that the non-etched regions of step (ii) are exposed,
(v) depositing microstructures on said non-etched regions, wherein the depositing of the microstructures is performed by electroplating, cathodic or anodic polarization or electrophoresis, preferably by electroplating,
**characterized in that**
in step (v) the deposition is stopped before the growth of the microstructures starts narrowing their cavities formed between adjacently growing microstructures, and further comprising the steps of
(vi) optionally washing the entire substrate having the microstructures deposited thereon with a solvent for removing the insulating material, and
(vii) repeating successively above steps (iii), (iv), (v) and (vi) in a loop process till a desired height of the microstructures is reached, where at least in the final loop the washing step (vi) is carried out.

2. The process according to claim 1, wherein the at least two regions of said heterogeneous polycrystalline substrate are different in crystal structure, crystal phase or chemical composition.

3. The process according to claim 1 or 2, comprising the steps of (i) providing a heterogeneous polycrystalline substrate having two or more phases, and (ii) conducting a selective etching process on said two-phase polycrystalline substrate, resulting in etched regions correspondent to one phase thereof and non-etched regions corresponding to the other phase.

4. The process according to any one of claims 1 to 3, wherein the substrate is a copper-zinc system having either two phases α and β or having a mixture of two crystal structures FCC (face-centered cubic) and BCC (body-centered cubic).

5. The process according to any one of claims 1 to 4, wherein in step (ii) the selective etching is carried out by wet etching by means of a solution of nitric acid.

6. The process according to any one of claims 1 to 5, wherein in step (iii) the insulating material is an organic resin, preferably applied on the entire surface of the substrate by means of coating.

7. The process according to any one of claims 1 to 6, wherein the electroplating time is less than 300 s.

8. The process according to any one of claims 1 to 7, wherein step (vii) is carried out as many times till an height of the microstructures in the range of 15 µm to 250 µm, preferably 100 µm to 250 µm, more preferably 100 µm to 200 µm, is reached.

9. Porous layer structure on a heterogeneous polycrystalline substrate, obtained by the process as defined in any one of claims 1 to 8.

10. Use of the porous layer structure on a heterogeneous polycrystalline substrate according to claim 9 in gas sensors or implant parts, or mechanical equipments.

## Patentansprüche

1. Verfahren zur Herstellung einer porösen Schichtstruktur auf einem heterogenen polykristallinen Substrat, umfassend die folgenden Schritte:
(i) das Bereitstellen eines heterogenen polykristallinen Substrats mit mindestens zwei Bereichen mit unterschiedlichen chemisch-physikalischen Eigenschaften,
(ii) das Durchführen eines selektiven Ätzverfahrens auf dem polykristallinen Substrat, resultierend in geätzten Bereichen, die einem der mindestens zwei Bereiche des Substrats entsprechen, und nicht-geätzten Bereichen, die dem anderen der mindestens zwei Bereiche des Substrats entsprechen,
(iii) das Bedecken der gesamten Oberfläche des Substrats mit einem Isolationsmaterial, wodurch eine Isolationsschicht auf dem Substrat bewirkt wird,
(iv) das Polieren der Oberfläche des Substrats zu einem solchen Ausmaß, daß die nicht-geätzten Bereiche von Schritt (ii) ausgesetzt werden,
(v) das Abscheiden von Mikrostrukturen auf den nicht-geätzten Bereichen, wobei das Abscheiden der Mikrostrukturen durch Elektroplattieren, kathodische oder anodische Polarisation oder Elektrophorese, vorzugsweise durch Elektroplattieren, durchgeführt wird,
**dadurch gekennzeichnet, daß**,
in Schritt (v) die Abscheidung gestoppt wird, bevor das Wachstum der Mikrostrukturen beginnt, daß sich deren Lücken bzw. Vertiefungen, gebildet zwischen angrenzend wachsenden Mikrostrukturen, verengen, und weiter umfassend die Schritte
(vi) gegebenenfalls des Waschens des gesamten Substrats mit den darauf abgeschiedenen Mikrostrukturen mit einem Lösungsmittel zum Entfernen des Isolationsmaterials, und
(vii) des Nacheinanderwiederholens der vorstehenden Schritte (iii), (iv), (v) und (vi) in einem Schleifenprozess, bis eine gewünschte Höhe der Mikrostrukturen erreicht ist, wobei mindestens in der finalen Schleife der Waschschritt (vi) durchgeführt wird.

2. Verfahren gemäß Anspruch 1, wobei die mindestens zwei Bereiche des heterogenen polykristallinen Substrats unterschiedlich in Kristallstruktur, Kristallphase oder chemischer Zusammensetzung sind.

3. Verfahren gemäß Anspruch 1 oder 2, umfassend den Schritt des (i) Bereitstellens eines heterogenen polykristallinen Substrats mit zwei oder mehr Phasen, und (ii) des Durchführens eines selektiven Ätzprozesses an dem zweiphasigen polykristallinen Substrat, resultierend in geätzten Bereichen, entsprechend der einen Phase davon, und nicht-geätzten Bereichen, entsprechend der anderen Phase.

4. Verfahren gemäß einem der Ansprüche 1 bis 3, wobei das Substrat ein Kupfer-Zink-System mit entweder zwei Phasen α und β oder mit einem Gemisch von zwei Kristallstrukturen FCC (flächenzentriert kubisch) und BCC (raumzentriert kubisch) ist.

5. Verfahren gemäß einem der Ansprüche 1 bis 7, wobei in Schritt (ii) das selektive Ätzen durch Naßätzen mittels einer Lösung von Salpetersäure durchgeführt wird.

6. Verfahren gemäß einem der Ansprüche 1 bis 5, wobei in Schritt (iii) das Isolationsmaterial ein organisches Harz ist, vorzugsweise aufgebracht auf der gesamten Oberfläche des Substrats mittels Beschichten.

7. Verfahren gemäß einem der Ansprüche 1 bis 6, wobei die Elektroplattierungsdauer weniger als 300 s beträgt.

8. Verfahren gemäß einem der Ansprüche 1 bis 7, wobei Schritt (vii) so viele Male durchgeführt wird, bis eine Höhe der Mikrostrukturen in dem Bereich von 15 µm bis 250 µm, vorzugsweise 100 µm bis 250 µm, mehr bevorzugt 100 µm bis 200 µm, erreicht wird.

9. Poröse Schichtstruktur auf einem heterogenen polykristallinen Substrat, erhalten durch das Verfahren, wie in einem der Ansprüche 1 bis 8 definiert.

10. Verwendung der porösen Schichtstruktur auf einem heterogenen polykristallinen Substrat gemäß Anspruch 9 in Gassensoren oder Implantatteilen oder mechanischen Ausrüstungen.

## Revendications

1. Procédé de production d'une structure de couche poreuse sur un substrat polycristallin hétérogène, comprenant les étapes suivantes :
(i) la fourniture d'un substrat polycristallin hétérogène comportant au moins deux régions avec des propriétés chimico-physiques différentes,
(ii) la réalisation d'un procédé de gravure sélective sur ledit substrat polycristallin, conduisant à des régions gravées correspondant à l'une des au moins deux régions dudit substrat et à des régions non gravées correspondant à l'autre desdites au moins deux régions dudit substrat,
(iii) le revêtement de la surface entière du substrat par un matériau isolant, en créant ainsi une couche isolante sur ledit substrat,
(iv) le polissage de la surface du substrat jusqu'à ce que les régions non gravées de l'étape (ii) soient exposées,
(v) le dépôt de microstructures sur lesdites régions non gravées, dans lequel le dépôt des microstructures est réalisé par dépôt électrolytique, polarisation cathodique ou anodique ou électrophorèse, de préférence par dépôt électrolytique,
**caractérisé en ce que**
à l'étape (v) le dépôt est arrêté avant que la croissance des microstructures ne commence à rétrécir leurs cavités formées entre des microstructures à croissance adjacente, et comprenant en outre les étapes de
(vi) lavage facultatif du substrat entier comportant les microstructures déposées au-dessus avec un solvant pour éliminer le matériau isolant, et
(vii) répétition successive des étapes (iii), (iv), (v) et (vi) ci-dessus dans un procédé en boucle jusqu'à ce qu'une hauteur souhaitée des microstructures soit atteinte, où au moins dans la boucle finale l'étape de lavage (vi) est réalisée.

2. Procédé selon la revendication 1, dans lequel les au moins deux régions dudit substrat polycristallin hétérogène sont différentes en termes de structure cristalline, de phase cristalline ou de composition chimique.

3. Procédé selon la revendication 1 ou 2, comprenant les étapes de (i) fourniture d'un substrat polycristallin hétérogène comportant deux phases ou plus, et (ii) réalisation d'un procédé de gravure sélective sur ledit substrat polycristallin à deux phases, conduisant à des régions gravées correspondant à une phase de celui-ci et à des régions non gravées correspondant à l'autre phase.

4. Procédé selon l'une quelconque des revendications 1 à 3, dans lequel le substrat est un système cuivre-zinc soit comportant deux phases α et β soit comportant un mélange de deux structures cristallines FCC (cubique à faces centrées) et BCC (cubique à corps centré).

5. Procédé selon l'une quelconque des revendications 1 à 4, dans lequel à l'étape (ii) la gravure sélective est réalisée par gravure humide au moyen d'une solution d'acide nitrique.

6. Procédé selon l'une quelconque des revendications 1 à 5, dans lequel à l'étape (iii) le matériau isolant est une résine organique, de préférence appliquée sur la surface entière du substrat au moyen d'un revêtement.

7. Procédé selon l'une quelconque des revendications 1 à 6, dans lequel la durée du dépôt électrolytique est moins de 300 s.

8. Procédé selon l'une quelconque des revendications 1 à 7, dans lequel l'étape (vii) est réalisée autant de fois jusqu'à ce qu'une hauteur des microstructures dans la plage de 15 *µ*m à 250 *µ*m, de préférence de 100 *µ*m à 250 *µ*m, de manière davantage préférée de 100 *µ*m à 200 *µ*m, soit atteinte.

9. Structure de couche poreuse sur un substrat polycristallin hétérogène, obtenue par le procédé tel que défini dans l'une quelconque des revendications 1 à 8.

10. Utilisation de la structure de couche poreuse sur un substrat polycristallin hétérogène selon la revendication 9 dans des capteurs de gaz ou des pièces d'implant, ou des équipements mécaniques.
